# EUROPÄISCHE PATENTANMELDUNG

(11) **EP 1 288 187 A2**
(43) Veröffentlichungstag der Anmeldung: **05.03.2003**
(21) Anmeldenummer: 02018144.2
(22) Anmeldetag: 14.08.2002
(51) Int. Cl.: C07C 67/24, C07C 69/007

(54) **Verfahren zur Herstellung von Carbonsäurebenzylestern**

(30) Priorität: 27.08.2001 DE 10141830
(71) Anmelder: Bayer Aktiengesellschaft, 51368 Leverkusen (DE)
(72) Erfinder: Ooms, Pieter, Dr., 47800 Krefeld (DE); Schenke, Bernd-Ulrich, 46236 Bottrop (DE); Stürmann, Martin, Dr., 51373 Leverkusen (DE)

(57) **Zusammenfassung**

Carbonsäurebenzylestern können durch Umsetzung von Dibenzylether mit Carbonsäuren und gegebenenfalls Carbonsäureanhydriden in Gegenwart einer oder mehrerer, bevorzugt einer Säure aufgebracht auf einem Träger, als Katalysator hergestellt werden.

## Beschreibung

Die vorliegende Erfindung betrifft ein Verfahren zur Herstellung von Carbonsäurebenzylestern durch Reaktion von Dibenzylether mit Carbonsäuren und gegebenenfalls Carbonsäureanhydriden im Molverhältnis 1: 1 bis 1: 50 bei 10 bis 200°C und Drucken im Bereich von 0,1 bis 50 bar in Gegenwart eines oder mehrerer, bevorzugt einer heterogenisierten Säure als Katalysator.

Benzylacetat, die Hauptkomponente des Jasminöls, ist ein wichtiger Riechstoff zur Herstellung von Duftkompositionen und Ausgangsprodukt für die Herstellung von Fruchtethern.

Die Herstellung von Benzylacetat durch Veresterung von Benzylalkohol mit Essigsäure ist seit langem bekannt.

Benzylacetat kann auch durch Umsetzung von Benzylchlorid mit Alkaliacetaten gegebenenfalls in Gegenwart von Phasentransferreagenzien hergestellt werden (Wang et al., Chem. Eng. Commun., 100, S.135 bis 147 (1991)). Nachteilig ist die Bildung von Salzen, die entsorgt werden müssen und somit die Wirtschaftlichkeit dieser Verfahren verringern.

DD-A5-286 577 beschreibt die Herstellung von Benzylacetat durch Umsetzung von Dibenzylether mit Essigsäureanhydrid. Nachteilig sind die drastischen Reaktionsbedingungen (300°C/20 MPa) und die nur mäßigen Ausbeuten.

Es bestand also die Aufgabe, ausgehend von Dibenzylether ein Verfahren zur Herstellung von Carbonsäurebenzylestern zu entwickeln, welches unter milden Reaktionsbedingungen durchführbar ist und kostengünstig zu guten Ausbeuten führt.

Es wurde ein Verfahren zur Herstellung von Carbonsäurebenzylestern der Formel 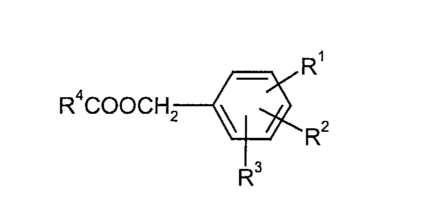 worin
- R¹ bis R³: gleich oder verschieden sind und für Wasserstoff, C₁-C₆-Alkyl, C₁-C₆-Alkoxy-, C₁-C₆-Halogenalkyl, C₁-C₆-Halogenalkoxy-, CN, CO(C₁-C₆-Alkyl), NO₂ oder Halogen und
- R⁴: für Wasserstoff, C₁-C₆-Alkyl, C₁-C₆-Alkenyl, C₁-C₁₂-Aryl, C₁-C₆-Halogenalkyl, C₁-C₆-Halogenalkenyl- oder C₁-C₁₂-Halogenaryl,
stehen,
aus Dibenzylethern gefunden, das dadurch gekennzeichnet ist dass Dibenzylether der Formel 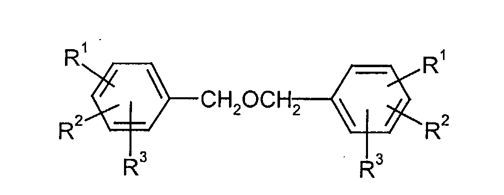 worin
R¹, R² und R³ die obengenannte Bedeutung haben,
oder Gemische aus Dibenzylethern und Benzylalkoholen der Formel 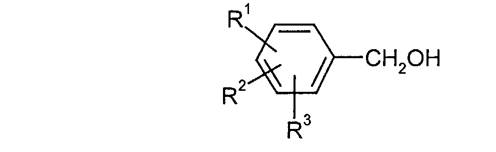 worin
R¹, R ² und R ³ die obengenannte Bedeutung haben
mit Carbonsäuren der Formel

R⁴COOH

worin
R⁴ die obengenannte Bedeutung hat,
in Gegenwart einer oder mehrerer auf einem Träger aufgebrachten Säure als Katalysator, umgesetzt werden.

Das erfindungsgemäße Verfahren lässt sich kostengünstig und unter milden Reaktionsbedingungen durchführen.

Die Reste R¹ bis R³ haben im allgemeinen die folgende Bedeutung:

Alkyl bedeutet im allgemeinen ein geradkettiger oder verzweigter Kohlenwasserstoffrest mit 1 bis 6, bevorzugt 1 bis 4, im besondere bevorzugt 1 oder 2 Kohlenstoffatomen. Beispielsweise seien Methyl, Ethyl, Propyl, iso-Propyl, Butyl, iso-Butyl, Pentyl, iso-Pentyl, Hexyl und iso-Hexyl genannt. Bevorzugt sind Methyl, Ethyl, Propyl, iso-Propyl, Butyl, Pentyl und Hexyl, im besondere bevorzugt sind Methyl und Ethyl.

Alkoxy bedeutet im allgemeinen ein geradkettiger oder verzweigter Alkoxyrest mit 1 bis 6, bevorzugt 1 bis 4, im besondere bevorzugt 1 oder 2 Kohlenstoffatomen. Beispielsweise seien Methoxy, Ethoxy, Propoxy, iso-Propoxy, Butoxy, iso-Butoxy, Pentoxy, iso-Pentoxy, Hexoxy und iso-Hexoxy genannt. Bevorzugt sind Methoxy, Ethoxy, Propoxy, iso-Propoxy, Butoxy, iso-Butoxy, Pentoxy und Hexoxy, im besondere bevorzugt sind Methoxy und Ethoxy.

Halogenalkyl bedeutet im allgemeinen ein geradkettiger oder verzweigter Kohlenwasserstoffrest mit 1 bis 6, bevorzugt 1 bis 4, im besondere bevorzugt 1 oder 2 Kohlenstoffatomen mit 1 bis 10, bevorzugt 1 bis 8, im besondere bevorzugt mit 1 bis 5 Halogenatomen. Beispielsweise seien Chlormethyl, Fluormethyl, Difluormethyl, Trifluormethyl, Fluorethyl, Fluorpropyl und Hexafluorbutyl genannt. Bevorzugt sind Fluormethyl, Difluormethyl, Trifluormethyl, Fluorethyl, Fluorpropyl und Hexafluorbutyl, im besondere bevorzugt sind Fluormethyl und Trifluormethyl.

Halogenalkoxy bedeutet im allgemeinen ein geradkettiger oder verzweigter Alkoxyrest mit 1 bis 6, bevorzugt 1 bis 4, im besondere bevorzugt 1 oder 2 Kohlenstoffatomen mit 1 bis 10, bevorzugt 1 bis 8, im besondere bevorzugt mit 1 bis 5 Halogenatomen. Beispielsweise seien Chlormethoxy, Fluormethoxy, Difluormethoxy, Trifluormethoxy, Fluorethoxy, Fluorpropoxy und Hexafluorbutoxy genannt. Bevorzugt sind Chlormethoxy, Fluormethoxy, Trifluormethoxy, Fluorethoxy, Fluorpropoxy und Hexafluorbutoxy, im besondere bevorzugt sind Fluormethoxy und Trifluormethoxy.

Halogen bedeutet im allgemeinen Fluor, Chlor, Brom und Jod, bevorzugt Fluor, Chlor und Brom, im besondere Fluor und Chlor.

Ganz besonders bevorzugte Substituenten für R¹ bis R³ sind Wasserstoff, Methyl, Trifluormethyl, Methoxy, Fluor oder Chlor.

Nach dem erfindungsgemäßen Verfahren können beispielsweise die folgenden Carbonsäurebenzylestern hergestellt werden: Ameisensäurebenzylester, Essigsäurebenzylester, Propionsäurebenzylester, Buttersäurebenzylester, Pentansäurebenzylester, Hexansäurebenzylester, Heptansäurebenzylester, Octansäurebenzylester, Nonansäurebenzylester, Decansäurebenzylester, Undecansäurebenzylester, Dodecansäurebenzylester, Tridecansäurebenzylester, Tetradecansäurebenzylester, Pentadecansäurebenzylester, Hexadecansäurebenzylester, Heptadecansäurebenzylester, Oktadecansäurebenzylester, Nonadecansäurebenzylester, 2-Hydroxybenzoesäurebenzylester, 3-Hydroxybenzoesäurebenzylester, 4-Hydroxybenzoesäurebenzylester, 3-Chlor-2-hydroxybenzoesäurebenzylester, 4-Chlor-2-hydroxybenzoesäurebenzylester, 5-Chlor-2-hydroxybenzoesäurebenzylester, 6-Chlor-2-hydroxybenzoesäurebenzylester, 3-Brom-2-hydroxybenzoesäurebenzylester, 3-Fluor-2-hydroxybenzoesäurebenzylester, 4-Fluor-2-hydroxybenzoesäurebenzylester, 2-Fluor-3-hydroxybenzoesäurebenzylester, 2-Fluor-4-hydroxybenzoesäurebenzylester, 3-Fluor-2-hydroxybenzoesäurebenzylester, 2-Fluor-5-hydroxybenzoesäurebenzylester, 2-Fluor-6-hydroxybenzoesäurebenzylester, 2-Hydroxy-3-methylbenzoesäurebenzylester, 2-Hydroxy-4-methylbenzoesäurebenzylester, 3-Hydroxy-2-methylbenzoesäurebenzylester, 4-Hydroxy-2-methylbenzoesäurebenzylester, 2-Fluor-6-hydroxy-4-methoxybenzoesäurebenzylester, 3-Trifluormethyl-2-hydroxybenzoesäurebenzylester, 4-Trifluormethyl-2-hydroxybenzoesäurebenzylester, 2-Trifluormethyl-3-hydroxybenzoesäurebenzylester, 2-Fluorethyl-4-hydroxybenzoesäurebenzylester und 4-Fluorbutyl-2-hydroxybenzoesäurebenzylester.

Bei dem im erfindungsgemäßen Verfahren eingesetzten Dibenzylether werden unsubstituierten oder substituierten Dibenzylether eingesetzt.

Besonders bevorzugt wird unsubstituierter Dibenzylether eingesetzt.

In das erfindungsgemäßen Verfahren können Dibenzylether oder Dibenzylether/Benzylalkoholgemische, wie sie beispielsweise bei der Herstellung von Benzylalkohol aus Benzylchlorid anfallen, eingesetzt werden. Der Gehalt an Dibenzylether kann 50 bis 100 %, bevorzugt 60 bis 99 %, besonders bevorzugt 70 bis 98 % sein.

Bei den im erfindungsgemäßen Verfahren eingesetzten Carbonsäuren handelt es sich um geradkettige und verzweigte, gesättigte und ungesättigte Alkyl-, Aralkyl- und Arylcarbonsäuren mit 1 bis 50 C-Atomen, wie beispielsweise Ameisensäure, Essigsäure, Propionsäure, Buttersäure, Isobuttersäure, Valeriansäure, Isovaleriansäure, Capronsäure, Heptansäure, Caprylsäure, Nonansäure, Caprinsäure, Undecansäure, Laurinsäure, Tridecansäure, Myristinsäure, Palmitinsäure, Stearinsäure, Ölsäure, Linolsäure, Chloressigsäure, Linolensäure, Acrylsäure, Methacrylsäure, Zimtsäure, Phenylessigsäure, Benzoesäure oder Salicylsäure. Bevorzugt sind Carbonsäuren mit 2 bis 30 C-Atomen, besonders bevorzugt 2 bis 10 C-Atomen. Ganz besonders bevorzugte Carbonsäuren sind Ameisensäure, Essigsäure, Chloressigsäure, Propionsäure und Hexansäure.

Das erfindungsgemäße Verfahren wird vorzugsweise unter Entfernung des gebildeten Wassers durchgeführt. Geeignet ist die Entfernung des Wassers durch Destillation oder durch Durchleiten eines inerten Gases wie beispielsweise Stickstoff. Bevorzugt werden zur Entfernung des gebildeten Wassers wasserentziehende Mittel eingesetzt, beispielsweise Zeolithe, Aluminiumoxide oder Tonerden. Besonders bevorzugt wird das gebildete Wasser dadurch entfernt, dass man die Umsetzung in Gegenwart des entsprechenden Anhydrids der eingesetzten Carbonsäure als wasserentziehendes Mittel durchführt. Ganz besonders bevorzugte Anhydride sind Essigsäureanhydrid, Chloressigsäureanhydrid, Propionsäureanhydrid und Benzoesäureanhydrid.

Im erfindungsgemäßen Verfahren werden vorzugsweise 2 bis 50 Äquivalente an Carbonsäure, bevorzugt 3 bis 30 Äquivalente, besonders bevorzugt 4 bis 20 Äquivalente, bezogen auf Dibenzylether, eingesetzt.

Wird das erfindungsgemäße Verfahren in Gegenwart des entsprechenden Anhydrids der eingesetzten Carbonsäure durchgeführt, so werden vorzugsweise 0,1 bis 10 Äquivalente Anhydrid, bevorzugt 0,5 bis 7,5 Äquivalente, besonders bevorzugt 1 bis 5 Äquivalente, bezogen auf Dibenzylether, eingesetzt. Da ein Molekül eingesetztes Anhydrid unter Wasseraufnahme zu 2 Molekülen Carbonsäure abreagiert, können im erfindungsgemäßen Verfahren geringere Mengen an Carbonsäure eingesetzt werden. Es werden dann vorzugsweise 1 bis 25 Äquivalente Carbonsäure, bevorzugt 1,5 bis 15 Äquivalente, besonders bevorzugt 2 bis 10 Äquivalente Carbonsäure, bezogen auf Dibenzylether, eingesetzt.

Geeignete Katalysatoren für das erfindungsgemäße Verfahren sind anorganische Säuren wie beispielsweise Schwefeltrioxid, Schwefelsäure, Chlorwasserstoff, Bromwasserstoff, Jodwasserstoff, Flusssäure, Perchlorsäure, Chlorsulfonsäure oder Phosphorsäure, organische Säuren wie beispielsweise Trifluoressigsäure, Methansulfonsäure, Ethansulfonsäure, Benzolsulfonsäure, 4-Toluolsulfonsäure oder Trifluormethansulfonsäure und Lewissäuren wie beispielsweise Bortrifluorid, Aluminiumchlorid, Aluminiumbromid, Aluminiumiodid, Zinkchlorid, Zinnchlorid, Titanchlorid oder Zirkonchlorid, aufgebracht auf einem oder mehreren, bevorzugt einem, Träger.

Bevorzugt sind Schwefeltrioxid, Schwefelsäure, Trifluormethansulfonsäure, 4-Toluolsulfonsäure und Bortrifluorid, aufgebracht auf Träger, besonders bevorzugt Schwefeltrioxid, Schwefelsäure, Trifluormethansulfonsäure und Bortrifluorid, aufgebracht auf einem Träger.

Geeignete Träger für das erfindungsgemäße Verfahren sind Oxide oder Sulfate von Elementen der Gruppen II A (Gruppe 2 nach IUPAC), beispielweise Magnesium, Calcium oder Barium, III B (Gruppe 3 nach IUPAC), beispielweise Scandium, Yttrium oder Lanthan, IV B (Gruppe 4 nach IUPAC), beispielsweise Titan, Zirkon oder Hafnium, V B (Gruppe 5 nach IUPAC), beispielsweise Niob oder Tantal, VII B (Gruppe 7 nach IUPAC), beispielsweise Mangan, VIII (Gruppen 8, 9 und 10 nach IUPAC), beispielsweise Eisen oder Nickel, III A (Gruppe 13 nach IUPAC), beispielsweise Al und IV A (Gruppe 14 nach IUPAC), beispielsweise Silizium, Germanium, Zinn oder Blei und Kohlenstoff.

Beispielsweise sind CaO, MgO, ZrO2, TiO₂, HfO₂, SnO₂, Al₂O₃, SiO₂, Al₂O₃.SiO₂ (Alumosilikate wie Zeolithe oder Schichtsilikate), Nb₂O₅, Ta₂O₅, Fe₂O₃, LaSO₄ oder CaSO₄ und Aktivkohlen zu nennen.

Bevorzugt sind CaO, MgO, ZrO₂, TiO₂, HfO₂, SnO₂, Al₂O₃.SiO₂, Al₂O₃, SiO₂, Nb₂O₅, Ta₂O₅, Fe₂O₃, LaSO₄ oder CaSO₄, im besondere bevorzugt sind CaO, MgO, SnO₂, ZrO₂, TiO₂, HfO₂, Al₂O₃, SiO₂, Al₂O₃.SiO₂, Nb₂O₅ und Ta₂O₅.

Ganz im besondere als Katalysatoren bevorzugt sind sulfatierte Oxide (Supersäure) wie SO₃ auf CaO, MgO, ZrO₂, TiO₂, HfO₂, SnO₂, Al₂O₃, SiO₂, Al₂O₃, SiO₂, Nb₂O₅, Ta₂O₅ oder Fe₂O₃.

Methoden zur Herstellung sind gut bekannt und beispielsweise beschrieben in Applied Catalysis A, 146, 1996, S. 3 bis 32, Catalysis Today 20, S. 219 bis 256 (1994) und WO 00/64849).

Die Säuren oder deren Hydrate, können auf einen Träger aufgebracht, gegebenenfalls kalziniert, als heterogener Katalysator eingesetzt werden.

Die Katalysatoren können z.B. als Pulver oder Formkörper eingesetzt werden und nach der Umsetzung z.B. durch Filtration, Sedimentation oder Zentrifugieren abgetrennt werden.

Für den Fall der Anordnung als Festbett werden die Säuren vorzugsweise auf einem Träger aufgebracht und als Formkörper, Z.B. als Kugeln, Zylinder, Stäbchen, Hohlzylinder, Ringe usw. eingesetzt.

Diese heterogenisierten Säuren werden gegebenenfalls durch Wärme, gegebenenfalls im Vakuum, gegebenenfalls durch Waschen mit hydrophilen organischen Flüssigkeiten wie beispielsweise die eingesetzte Carbonsäure oder das eingesetzte Carbonsäureanhydrid oder gegebenenfalls durch azeotrope Destillation mit organischen Flüssigkeiten wie Toluol, Xylol oder Methylenchlorid getrocknet.

Die geträgerten Säuren werden bei Arbeiten mit suspendiertem Katalysatoren in Rührgefäßen in Mengen von 0,1 bis 100 Gew.-%, bevorzugt von 0,5 bis 90 Gew.-% und besonders bevorzugt von 1,0 bis 80 Gew.-%, bezogen auf Dibenzylether, verwendet.

Im Falle einer kontinuierlichen Fahrweise im Gegen- oder Gleichstrom oder in der Rieselphase am Festbettkatalysator werden Katalysatorbelastungen von 0,05 g bis 5000 g Dibenzylether pro Liter immobilisierten Säure pro Stunde, bevorzugt von 0,1 bis 4000 g / 1.h und besonders bevorzugt von 1,0 bis 3000 g / 1.h verwendet.

Vorzugsweise wird das erfindungsgemäße Verfahren unter intensiver Durchmischung der Reaktionspartner durchgeführt. Eine intensive Durchmischung kann auf verschiedene, dem Fachmann bekannte Weisen, etwa durch Rührern, Düsen, Strombrecher, statische Mischer, Pumpen, turbulente Strömungen in engen Röhren und durch Ultraschall erreicht werden.

Derartige Vorrichtungen sind in Ullmann's Encyclopedia of Industrial Chemistry, 5. Auflage, Band B, Unit Operations, Abschnitte 25, 26, B4 S. 569 bis 570, Verlag Chemie, Weinheim 1988, näher beschrieben.

Eine bevorzugte Ausführungsform des erfindungsgemäßen Verfahrens ist indem man in eine Mischung oder Suspension der geträgerte Säure und Carbonsäure und/oder Carbonsäureanhydrid Dibenzylether zugibt und nach Beendigung der Reaktion den Katalysator durch z.B. Filtration oder Zentrifugieren abtrennt.

Eine weitere bevorzugte Durchführungsform ist das Gleichstromverfahren, bei der Dibenzylether und Carbonsäure und/oder Carbonsäureanhydrid im Gleichstrom beispielsweise von oben her auf eine in einem Rohr angeordnete Katalysatorschüttung aufgebracht und unten am Fuß des Rohres Carbonsäurebenzylester abgezogen werden.

In einer weiteren bevorzugten Ausführungsform des erfindungsgemäßen Verfahrens wird dieses in der Rieselphase durchgeführt und die geträgerte Säure liegt als Festbettkatalysator vor. Bevorzugt befindet sich die Katalysatorschüttung in einem senkrecht stehenden Rohrreaktor, welcher vorzugsweise Zwischenböden zur besseren Verteilung des Flüssigkeitsstroms und zur besseren Benetzung der Katalysatorschüttung enthält.

Die Aufarbeitung kann sowohl im Falle des suspendierten Katalysators als auch bei Festbettverfahrensvariante so durchgeführt werden, dass ein mit Wasser nicht mischbares Lösungsmittel, vorzugsweise Toluol, zu den Reaktionsprodukten gegeben wird. Nach der Abtrennung der organischen Phase, welche den rohen Carbonsäurebenzylester enthält, kann diese beispielsweise durch Destillation weiter gereinigt werden.

Das erfindungsgemäße Verfahren kann diskontinuierlich, kontinuierlich oder teilkontinuierlich durchgeführt werden.

Die Temperatur, bei der das erfindungsgemäße Verfahren durchgeführt wird, beträgt vorzugsweise 15 bis 200, besonders bevorzugt 25 bis 190, ganz besonders bevorzugt 30 bis 180°C.

Bei der Durchführung der Reaktion oberhalb 115°C muss entsprechend dem Dampfdruck unter erhöhtem Druck gearbeitet werden. Der benötigte Überdruck ist dann wenigstens gleich dem Dampfdruck des Reaktionsgemisches. Es kann bis etwa 50 bar betragen, vorzugsweise bis 25 bar.

Gegebenenfalls kann das erfindungsgemäße Verfahren unter einem üblichen Schutzgas wie beispielsweise Stickstoff, Helium oder Argon, durchgeführt werden.

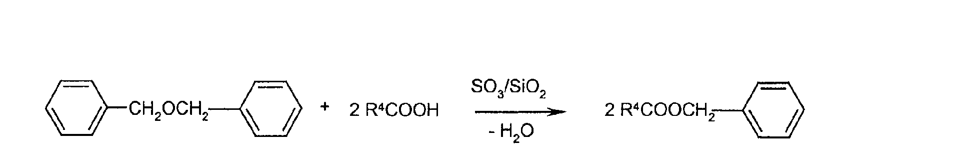

Nach dem erfindungsgemäßen Verfahren erhält man Carbonsäurebenzylester in guten Ausbeuten bei hohem Umsatz und guter Selektivität. Das erfindungsgemäße Verfahren ist ohne hohen apparativen Aufwand einfach durchführbar.

### Beispiele

Die Prozentangaben der nachfolgenden Beispiele beziehen sich auf das Gewicht.

### Beispiel 1

99,2 g (0,5 Mol) Dibenzylether, 120,0 g (2,0 Mol) Essigsäure und 1,0 g eines sulfatierten Kieselgels (15 % SO₃ / 1 SiO₂) wurden in einem Kolben mit Strombrecher und Flügelrührer unter kräftigem Rühren (250 U/min) und unter Stickstoff bei 120°C erhitzt. Nach 7 Stunden Reaktionszeit wurde rasch abgekühlt, die organische Phase nach Zugabe von Toluol und Wasser abgetrennt und gaschromatographisch analysiert. Das Reaktionsgemisch enthielt Benzylacetat und Dibenzylether im Verhältnis 30 zu 65.

### Beispiel 2

Beispiel 2 wurde analog zu Beispiel 1 durchgeführt. Es wurden 300,3 g (5,0 Mol) Essigsäure und 3,0 g eines sulfatierten Alumosilikats (15 % SO₃ / 1 Al₂O₃.SiO₂) eingesetzt. Die Reaktionszeit betrug 7 Stunden. Das Reaktionsgemisch enthielt Benzylacetat und Dibenzylether im Verhältnis 42 zu 54.

### Beispiel 3

Beispiel 3 wurde analog zu Beispiel 1 durchgeführt. Es wurden 300,3 g (5,0 Mol) Essigsäure und 3,0 g eines sulfatierten Kieselgels (15 % SO₃ / 1 SiO₂) eingesetzt. Die Reaktionszeit betrug 7 Stunden. Das Reaktionsgemisch enthielt Benzylacetat und Dibenzylether im Verhältnis 53 zu 41.

### Beispiel 4

Beispiel 4 wurde analog zu Beispiel 1 durchgeführt. Es wurden 300,3 g (5,0 Mol) Essigsäure und 3,0 g eines sulfatierten Kieselgels (41 % SO₃ /l SiO₂) eingesetzt. Die Reaktionszeit betrug 5 Stunden. Das Reaktionsgemisch enthielt Benzylacetat und Dibenzylether im Verhältnis 70 zu 22.

### Beispiel 5

Beispiel 1 wurde wiederholt, aber mit 30,0 g (0,5 Mol) Essigsäure, 51,0 g (0,5 Mol) Essigsäureanhydrid und 3,0 g eines sulfatierten Nioboxids (75 g SO₃ / 1 Nb₂O₅) bei 100°C. Die Reaktionszeit betrug 7 Stunden. Das Reaktionsgemisch enthielt Benzylacetat und Dibenzylether im Verhältnis 45 zu 42.

### Beispiel 6

Beispiel 5 wurde wiederholt, aber mit 3,0 g eines sulfatierten Aluminiumoxids SPH 501 der Firma Rhone-Poulenc (15 % SO₃ / l Al₂O₃). Die Reaktionszeit betrug 5 Stunden. Das Reaktionsgemisch enthielt Benzylacetat und Dibenzylether im Verhältnis 63 zu 26.

### Beispiel 7

Beispiel 5 wurde wiederholt, aber mit 3,0 g eines sulfatierten Calciumsulfats (17 % SO₃ / l CaSO₄). Die Reaktionszeit betrug 1 Stunde. Das Reaktionsgemisch enthielt Benzylacetat und Dibenzylether im Verhältnis 75 zu 12.

### Beispiel 8

Beispiel 5 wurde wiederholt, aber mit 3,0 g eines sulfatierten Alumosilikats (15 % SO₃ / l Al₂O₃.SiO₂). Die Reaktionszeit betrug 3 Stunden. Das Reaktionsgemisch enthielt Benzylacetat und Dibenzylether im Verhältnis 89 zu 3.

### Beispiel 9

Beispiel 5 wurde wiederholt, aber mit 3,0 g eines sulfatierten Aluminiumoxids SPH 501 der Firma Rhone-Poulenc (15 % SO₃ / l Al₂O₃). Die Reaktionszeit betrug 5 Stunden. Das Reaktionsgemisch enthielt Benzylacetat und Dibenzylether im Verhältnis 63 zu 26.

### Beispiel 10

Beispiel 5 wurde wiederholt, aber mit 3,0 g eines sulfatierten Tantaloxids (15 % SO₃ / 1 Ta₂O₅). Die Reaktionszeit betrug 5 Stunden. Das Reaktionsgemisch enthielt Benzylacetat und Dibenzylether im Verhältnis 86 zu 5.

### Beispiel 11

Beispiel 5 wurde wiederholt, aber mit 3,0 g eines sulfatierten Kieselgels (41 % SO₃ /1 SiO₂). Die Reaktionszeit betrug 15 Minuten. Das Reaktionsgemisch enthielt Benzylacetat und Dibenzylether im Verhältnis 86 zu 5.

### Beispiel 12

Beispiel 5 wurde wiederholt, aber mit 0,5 g eines sulfatierten Titanoxids (Firma Aldrich). Die Reaktionszeit betrug 7 Stunden. Das Reaktionsgemisch enthielt Benzylacetat und Dibenzylether im Verhältnis 51 zu 35.

### Beispiel 13

Beispiel 5 wurde wiederholt, aber mit 0,5 g eines sulfatierten Zirkonoxids (Firma Acros). Die Reaktionszeit betrug 5 Stunden. Das Reaktionsgemisch enthielt Benzylacetat und Dibenzylether im Verhältnis 71 zu 14.

### Beispiel 14

Beispiel 5 wurde wiederholt, aber mit 3,0 g eines Katalysators, hergestellt durch Aufbringen von 20 g Trifluormethansulfonsäure auf 1000 ml Kieselgel.
Die Reaktionszeit betrug 3 Stunden. Das Reaktionsgemisch enthielt Benzylacetat und Dibenzylether im Verhältnis 83 zu 5.

### Beispiel 15

Beispiel 5 wurde wiederholt, aber mit 37,0 g (0,5 Mol) Propionsäure, 65,1 g (0,5 Mol) Propionsäureanhydrid und 3,0 g eines sulfatierten Kieselgels (15 % SO₃ /1 SiO₂). Die Reaktionszeit betrug 7 Stunden. Das Reaktionsgemisch enthielt Benzylpropionat und Dibenzylether im Verhältnis 76 zu 12.

### Beispiel 16 (Aufarbeitung)

Beispiel 5 wurde wiederholt, aber mit 3,0 g eines sulfatierten Kieselgels (41 % SO₃ /1 SiO₂) und nach 7 Stunden Laufzeit aufgearbeitet. Nach Filtration und destillativer Auftrennung des Reaktionsgemisches isoliert man bei 57 - 60°C / 0,25 mbar 106 g (70 %) Benzylacetat mit einer Reinheit von 97,5 %. Vor- und Nachlauf enthalten noch 7,4 g (5 %) Benzylacetat.

## Patentansprüche

1. Verfahren zur Herstellung von Carbonsäurebenzylestern der Formel 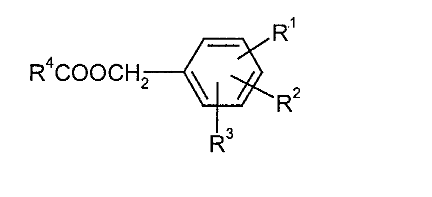 worin
R¹ bis R³ gleich oder verschieden sind und für C₁-C₆-Alkyl, C₁-C₆-Alkoxy-, C₁-C₆-Halogenalkyl, C₁-C₆-Halogenalkoxy-, CN, CO(C₁-C₆-Alkyl), NO₂ oder Halogen und
R⁴ für Wasserstoff, C₁-C₆-Alkyl, C₁-C₆-Alkenyl, C₁-C₁₂-Aryl, C₁-C₆-Halogenalkyl, C₁-C₆-Halogenalkenyl- oder C₁-C₁₂-Halogenaryl, stehen,
aus Dibenzylethern, **dadurch gekennzeichnet, dass** man Dibenzylether der Formel 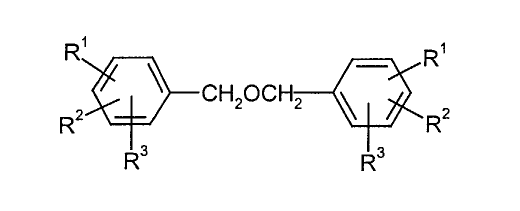 worin
R¹, R² und R³ die obengenannte Bedeutung haben,
oder Gemische aus Dibenzylethern und Benzylalkoholen der Formel 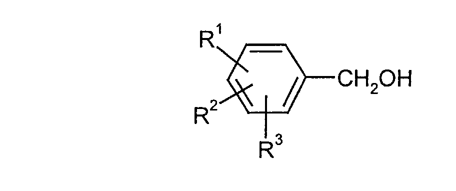 worin
R¹, R ² und R ³ die obengenannte Bedeutung haben
mit Carbonsäuren der Formel
R⁴COOH
worin
R⁴ die obengenannte Bedeutung hat,
in Gegenwart einer oder mehrerer, bevorzugt einer auf einem Träger aufgebrachten Säure als Katalysator, umsetzt.

2. Verfahren nach Anspruch 1, **dadurch gekennzeichnet, dass** die Säuren sowohl anorganische Säuren, organische Säuren oder Lewissäuren mit einem pH-Wert von 1 bis 6 sind.

3. Verfahren nach den Ansprüchen 1 und 2, **dadurch gekennzeichnet, dass** die Säuren Schwefeltrioxid, Schwefelsäure, Chlorwasserstoff, Bromwasserstoff, Jodwasserstoff, Flusssäure, Perchlorsäure, Chlorsulfonsäure, Phosphorsäure, Trifluoressigsäure, Methansulfonsäure, Ethansulfonsäure, Benzolsulfonsäure, 4-Toluolsulfonsäure, Bortrifluorid, Aluminiumchlorid, Aluminiumbromid, Aluminiumiodid, Zinkchlorid, Zinnchlorid, Titanchlorid oder Zirkonchlorid sind, aufgebracht auf einem oder mehreren, bevorzugt einem, Träger.

4. Verfahren nach den Ansprüchen 1 bis 3, **dadurch gekennzeichnet, dass** die Träger Oxide oder Sulfate von Elementen der Gruppen II A (Gruppe 2 nach IUPAC), III B (Gruppe 3 nach IUPAC), IV B (Gruppe 4 nach IUPAC), V B (Gruppe 5 nach IUPAC), VII B (Gruppe 7 nach IUPAC), VIII (Gruppe 8, 9 und 10 nach IUPAC), III A (Gruppe 13 nach IUPAC) und IV A (Gruppe 14 nach IUPAC) sind.

5. Verfahren nach den Ansprüchen 1 bis 4, **dadurch gekennzeichnet, dass** als Katalysatoren Schwefeltrioxid, Schwefelsäure und Trifluormethansulfonsäure auf Aktivkohle, CaO, MgO, ZrO₂, TiO₂, HfO₂, SnO₂, Al₂O₃, SiO₂, Al₂O₃.SiO₂ (Alumosilikate wie Zeolithe oder Schichtsilikate), Nb₂O_{5,} Ta₂O₅, Fe₂O₃, LaSO₄ oder CaSO₄ eingesetzt werden.

6. Verfahren nach den Ansprüchen 1 bis 5, **dadurch gekennzeichnet, dass** als Katalysatoren sulfatierte Metalloxide eingesetzt werden.

7. Verfahren gemäß einem oder mehreren der vorangegangenen Ansprüche 1 bis 6, **dadurch gekennzeichnet, dass** es sich bei dem Dibenzylether um unsubstituierten Dibenzylether handelt.

8. Verfahren gemäß einem oder mehreren der vorangegangenen Ansprüche 1 bis 7 **dadurch gekennzeichnet, dass** es sich bei dem Dibenzylether um einem substituierten Dibenzylether handelt, welcher einen oder mehrere Substituenten aus der Reihe C₁-C₆-Alkyl, C₁-C₆-Alkoxy-, CN, CO(C₁-C₆-Alkyl), NO₂ oder Halogen trägt.

9. Verfahren gemäß einem oder mehreren der vorangegangenen Ansprüche 1 bis 8, **dadurch gekennzeichnet, dass** 2 bis 50 Äquivalente an Carbonsäure, bezogen auf Dibenzylether, eingesetzt werden.

10. Verfahren gemäß einem oder mehreren der vorangegangenen Ansprüche 1 bis 9, **dadurch gekennzeichnet, dass** die Umsetzung in Gegenwart von wasserentziehenden Mitteln stattfindet.

11. Verfahren gemäß einem oder mehreren der vorangegangenen Ansprüche 1 bis 10, **dadurch gekennzeichnet, dass** die Umsetzung unter Entfernung von Wasser durch Destillation oder Durchleiten von Stickstoff stattfindet.

12. Verfahren gemäß einem oder mehreren der vorangegangenen Ansprüche 1 bis 11, **dadurch gekennzeichnet, dass** die Umsetzung in Gegenwart des entsprechenden Anhydrids der eingesetzten Carbonsäure durchgeführt wird.

13. Verfahren gemäß Anspruch 12, **dadurch gekennzeichnet, dass** 0,1 bis 10 Äquivalente Anhydrid, bezogen auf Dibenzylether eingesetzt werden.

14. Verfahren gemäß einem oder mehreren der vorangegangenen Ansprüche 1 bis 13, **dadurch gekennzeichnet, dass** die Umsetzung bei einer Temperatur von 15 bis 200°C durchgeführt wird.

15. Verfahren gemäß einem oder mehreren der vorangegangenen Ansprüche 1 bis 14, **dadurch gekennzeichnet dass** eine oder mehrere, geträgerte Säuren in einer Menge von 0,5 bis 100 Gew.-%, bezogen auf die Menge des Dibenzylethers, bei suspendiertem Katalysator bzw. mit Belastungen von 1,0 bis 3000 g Dibenzylether pro Liter heterogenisierter Supersäure pro Stunde bei der Anordnung als Festbettkatalysator eingesetzt wird.
